# EUROPEAN PATENT APPLICATION

(11) **EP 2 383 322 A1**
(43) Date of publication of application: **02.11.2011**
(21) Application number: 09834749.5
(22) Date of filing: 16.12.2009
(51) Int. Cl.: C09K 11/06

(54) **FLUORESCENT DYE-CONTAINING PARTICLES AND MANUFACTURING METHOD THEREOF**

(30) Priority: 25.12.2008 JP 2008330953
(71) Applicant: Nippon Sheet Glass Company Limited, Tokyo 108-6321 (JP)
(72) Inventor: MIKAMI, Shinji, Tokyo 108-6321 (JP)
(74) Representative: Hart-Davis, Jason
(86) International application number: PCT/JP2009/070964
(87) International publication number: WO 2010/073946

(57) **Abstract**

The fluorescent dye-containing particle of the present invention includes a porous matrix composed mainly of metal oxide, an organic fluorescent dye contained in the porous matrix, and a water-soluble polymer contained in the porous matrix. The fluorescent dye-containing particle can be produced by the steps of: preparing a dye-containing sol solution containing a sol solution obtained by hydrolysis of a metal compound, an organic fluorescent dye, and a water-soluble polymer; and forming a particle from the dye-containing sol solution by spray drying process.

## Description

### TECHNICAL FIELD

The present invention relates to a fluorescent dye-containing particle and a method for producing the fluorescent dye-containing particle.

### BACKGROUND ART

Particles in which a silica matrix contains an organic fluorescent dye are conventionally known. The organic fluorescent dye is introduced into the silica matrix by sol-gel process since the organic fluorescent dye has low heat resistance. However, such silica matrix produced by sol-gel process is porous, and thus there is a disadvantage that the organic fluorescent dye is easily eluted from the silica matrix.

As described in JP 2003-270154 A, a method of incorporating a dye into a matrix by allowing the dye to be bonded to chemically modified silicon is effective in improving the resistance to elution, the dispersibility of the dye and the emission intensity. However, dyes to be used are limited, and further the production cost increases.

JP 2007-99774 A describes a cosmetic composition of fluorescent particles containing the molecules of at least one fluorescent inorganic compound that are encapsulated in a matrix that is formed at least partially of at least one metal oxide in a physiologically acceptable medium and that contains an organic group (for example, phenyl or alkyl) capable of causing interaction with fluorescent organic molecules, for example, through van der Waals force or hydrogen bond.

### CITATION LIST

### Patent Literature

Patent Literature 1: JP 2003-270154 A
Patent Literature 2: JP 2007-99774 A

### SUMMARY OF INVENTION

### Technical Problem

As described above, various methods for improving the resistance to elution of organic fluorescent dye-containing particles have been proposed conventionally, which however have not necessarily yielded sufficient results. It is an object of the present invention to improve the resistance to elution of fluorescent dye-containing particles while avoiding an increase in production cost and a decrease in emission intensity.

### Solution to Problem

As a result of diligent studies on fluorescent dye-containing particles produced by sol-gel process, the inventor has found that it is effective, in suppressing elution of an organic fluorescent dye from a porous matrix, to allow the porous matrix to contain a water-soluble polymer. Thus, the present invention has been accomplished. That is, the present invention provides a fluorescent dye-containing particle including a porous matrix composed mainly of a metal oxide, an organic fluorescent dye contained in the porous matrix, and a water-soluble polymer contained in the porous matrix.

According to another aspect, the present invention provides a method for producing a fluorescent dye-containing particle. The method includes the steps of: preparing a dye-containing sol solution that includes a sol solution obtained by hydrolysis of a metal compound, an organic fluorescent dye and a water-soluble polymer; and forming a fluorescent dye-containing particle from the dye-containing sol solution by spray drying process.

### Advantageous Effects of Invention

Although the mechanism that the water-soluble polymer contributes to the improvement of the resistance to elution is not necessarily clear, the inventor considers it as follows. First, the water-soluble polymer is uniformly dispersed in a sol solution for forming a porous matrix because of its polarity. Accordingly, the water-soluble polymer is uniformly dispersed also in a gel formed using the sol solution, that is, in a porous matrix without being condensed. The size of the water-soluble polymer herein is far larger than that of the metal atoms that form the porous matrix and that of the dye molecules. Therefore, the water-soluble polymer has a function to fill the pores of the porous matrix. As a result, the sites where the dye molecules can contact a liquid such as water and alcohol decrease, and thus the number of the dye molecules that can easily leave the porous matrix also decreases.

Further, as shown in the examples to be mentioned later, the emission intensity also is improved by allowing the water-soluble polymer to be included in the porous matrix. Although the mechanism that the emission intensity is improved also is not necessarily clear, the emission intensity strongly depends on the dispersibility of the organic fluorescent dye in the porous matrix. From the result that the emission intensity has been improved, it can be inferred that the water-soluble polymer has contributed to the improvement of the dispersibility of the organic fluorescent dye.

The fluorescent dye-containing particle of the present invention can be produced by sol-gel process. In the sol-gel process, a hydrolyzable metal compound is used as a main raw material. The use of spray drying process for drying and particle formation enables a particle with a desired particle size to be produced without grinding step and classification step. The use of a metal compound having a hydrophobic organic group bonded to the metal element enhances the stability of the sol solution. Thus, gelation does not easily occur, even when the sol solution is left standing at room temperature.

### DESCRIPTION OF EMBODIMENTS

The method for producing a fluorescent dye-containing particle according to this embodiment employs a sol-gel process, and generally includes the following steps (1) to (3). Hereinafter, each step is described in detail.

(1) Preparation of dye-containing sol solution
(2) Formation of particles using dye-containing sol solution
(3) Particle drying (if necessary)

### (1) Preparation of dye-containing sol solution

The step for preparing a dye-containing sol solution can be performed at separate steps of preparing a sol solution and dispersing an organic fluorescent dye and a water-soluble polymer in the sol solution.

The organic fluorescent dye dispersed in a sol solution is easily degraded as compared to that stored in a solid state. Accordingly, the organic fluorescent dye may be added to the sol solution immediately before particles are formed by spray drying process. This can prevent the degradation of the organic fluorescent dye as much as possible. The water-soluble polymer can be added directly to the sol solution, which however takes time to dissolve therein in many cases. Accordingly, after the water-soluble polymer is dissolved in another solvent in advance, the preliminary solution may be added to the sol solution.

### (1-1) Preparation of sol solution

The sol solution can be prepared by hydrolysis of a metal compound in the presence of water and an acid catalyst. An acidic sol solution is more suitable for producing particles because the change from sol to gel proceeds mildly, as compared to an alkaline sol solution.

Typical examples of the hydrolyzable metal compound include organic metal compounds such as metal alkoxide, metal acetyl acetonate and metal carboxylate. Above all, metal alkoxide can be used suitably. Metal alkoxide has advantages of ease of availability, stability at normal temperature and normal pressure, less toxicity, and the like. Further, optical absorption does not occur in the visible light region, and thus the light emission from the organic fluorescent dye can be prevented from being absorbed into the particle matrix.

Metal alkoxide needs only to include a metal element M and at least one OR group bonded to the metal element M. An example of metal alkoxide to be used can be expressed by the general formula:

R¹ₘ,M(OR²)ₙ,

where the metal element M can be one selected from the group consisting of silicon, aluminium, titanium, zirconium and tantalum; n is generally an integer of 1 to 5; m is a natural number of 0 to 4; m = 0 means that the R¹ group is not included therein;
(m + n) is equal to the valence of the metal element M; R² is preferably an alkyl group, more preferably an alkyl group having 1 to 5 carbon atoms, which for example is a methyl group, an ethyl group, a propyl group and an isopropyl group; and R² can be a non-hydrolyzable hydrophobic organic group.

Examples of metal alkoxide include silicon alkoxide, aluminium alkoxide and titanium alkoxide. Examples of silicon alkoxide include tetramethoxysilane, tetraethoxysilane and methyltrimethoxysilane. Examples of aluminium alkoxide include aluminium isopropoxide and aluminium ethoxide. Examples of titanium alkoxide include isopropyl orthotitanate. Furthermore, one metal alkoxide may be used by itself, or two or more kinds of metal alkoxide may be used in combination.

In the case where the raw material for the sol solution used therein is a metal alkoxide that does not satisfy m = 0 in the above-mentioned general formula, that is, a metal alkoxide having a non-hydrolyzable hydrophobic organic group R¹, the hydrolysis and polymerization reactions proceed mildly. Thus, gelation of the sol solution does not occur easily. High stability of the sol solution allows easy handling of the sol solution.

The porous matrix of the particles produced by the method of this embodiment is composed mainly of a metal oxide. A typical example of the metal oxide is silica. That is, a typical example of the metal alkoxide to be used as the raw material for the sol solution is silicon alkoxide. Furthermore, when the raw material for the sol solution used therein is silicon alkoxide having a hydrophobic organic group in its molecule, e.g. methyltrimethoxysilane, the thus produced particles include the hydrophobic organic group bonded (specifically, covalently bonded) to silicon of which the porous matrix is composed. The hydrophobic organic group exerts some effect on the improvement of the resistance to elution because it increases the hydrophobicity of the particle surface. In addition, the hydrophobic organic group enables control of the properties of the particle surface. This is effective in using the particles dispersed in a resin or the like.

The hydrophobic organic group R¹ bonded to silicon can be at least one selected from the group consisting of alkyl group, phenyl group, alkyl halide group, vinyl group and glycidoxy group. These functional groups are chemically stable and thus are preferable.

The alkyl group to serve as the hydrophobic organic group R¹, for example, is at least one selected from the group consisting of methyl group, ethyl group and propyl group. The silicon alkoxide including silicon to which such a hydrophobic organic group is bonded is preferable because it is easily available and thus does not cause an increase in production cost.

The abundance ratio of the alkyl group with respect to silicon is suitably in the range of 10 to 50 mol%. This abundance ratio can be adjusted by the used ratio of the silicon alkoxide having an alkyl group (the hydrophobic organic group R¹) in its molecule. When the abundance ratio of the alkyl group is adjusted within this range, the hydrophobic effect of the alkyl group can be sufficiently exerted on the surface of the particles, and thus further improvement in the resistance to elution can be expected. In addition, the formation of the framework of the silica matrix is not impaired.

The presence of the hydrophobic organic group bonded to the metal element can be examined by a chemical analysis such as infrared absorption spectroscopy, nuclear magnetic resonance and gas chromatography. For example, whether or not the methyl group is bonded to silicon can be examined by Gas Chromatography-Mass Spectrometry (GC-MS) utilizing the fact that the decomposition temperature of the methyl group bonded to silicon is higher than the decomposition temperature of the organic fluorescent dye or the surfactant, and a quantitative analysis thereof also is possible.

The solvent to be used in the sol-gel process for dissolving the metal compound therein needs only to be capable of forming a homogeneous solvent with water. Examples thereof include alcohol, ketone, ester, ethylene glycol monoethyl ether (hereinafter, abbreviated as cellosolve) and glycol having no hydroxyl group at both ends. Examples of alcohol to be used include methanol, ethanol and isopropanol. Examples of ketone to be used include acetone and acetyl acetone. Examples of ester to be used include methyl acetate, ethyl acetate and propyl acetate. Examples of cellosolve to be used include ethyl cellosolve and butyl cellosolve. Examples of glycol to be used include propylene glycol and hexylene glycol.

The acid catalyst to be used for hydrolysis is not specifically limited. However, protonic acid can be used suitably. Specific examples thereof include nitric acid, hydrochloric acid, acetic acid, trifluoroacetic acid, trichloroacetic acid, citric acid, sulfuric acid, phosphoric acid and p-toluenesulfonic acid. Above all, acetic acid is suitable because of its high safety and ease of volatilization by drying. The acid catalyst may as well have been dissolved in water to be used for hydrolysis of the metal compound.

In this description, elements to be classified as a nonmetal element, such as silicon, also are regarded as a metal, in accordance with common practice in the field of sol-gel processing. The phrase "composed mainly of" means that the mass content of the mentioned component is highest.

### (1-2) Dispersion of water-soluble polymer and organic fluorescent dye in sol solution

The water-soluble polymer and the organic fluorescent dye are dispersed in the sol solution that has been prepared by the above-mentioned procedure.

The water-soluble polymer is a polymer that can be dissolved in water or a solvent containing water (e.g. a water-alcohol mixed solvent). Examples of the water-soluble polymer include polyvinylpyrrolidone, polyvinyl alcohol, celluloses (cellulose and its derivatives), chitosan, acrylamide water-soluble polymers (water-soluble polymers using acrylamide), carboxymethyl cellulose, dextrin, starch, polyethylene glycol, hydroxypropyl cellulose, pectin, guar gum, xanthan gum, tamarind gum, vinyl acetate-vinylpyrrolidone copolymer and vinylpyrrolidone-dimethylaminopropylmethacrylamide copolymer. One of these water-soluble polymers or two or more of them can be used. Further, these various water-soluble polymers are commercially available and can be obtained easily.

The water-soluble polymer is preferably one that is not hydrolyzed, or difficult to be hydrolyzed, under acid conditions or alkali conditions. This is because the effect of improving the resistance to elution might be reduced when the hydrolysis decreases the molecular weight. On the other hand, it also is possible to use a water-soluble polymer in which polymerization proceeds under acid conditions or alkali conditions. However, it is not preferable in view of the stability of the sol solution because the polymerization might cause an increase in concentration of the sol solution or precipitation of the water-soluble polymer in the sol solution.
Furthermore, it also is possible to use a water-soluble polymer that is reactive with silanol. However, similarly to above, the reaction might cause an increase in concentration of the sol solution or precipitation of the reaction product in the sol solution. Nevertheless, there are some methods, such as quickly performing the operation from the preparation of sol solution to the formation of particles by spray drying process, and filtering the precipitates, which can prevent the emergence of various problems resulting from the properties of the water-soluble polymer. As a water-soluble polymer that is stable even in the sol solution, polyvinylpyrrolidone and vinyl acetate-vinylpyrrolidone copolymer can be mentioned.

The amount of the water-soluble polymer to be added to the sol solution may be adjusted corresponding to the type and properties of the water-soluble polymer. Specifically, the amount of the water-soluble polymer to be added to the sol solution may be adjusted so that the content of the water-soluble polymer in the particles should be in the range of 1 to 30 mass%. When the water-soluble polymer is contained therein with such a content, the effect of improving the resistance to elution can be obtained without impairing other properties such as emission intensity.

Next, the organic fluorescent dye to be used may be a dye that is soluble in water or alcohol. Such an organic fluorescent dye is easily dispersed in the sol solution. Specific examples of the organic fluorescent dye include fluorescein dye, pyrazine dye, coumarin dye, naphthalimide dye, triazine dye, oxazine dye, dioxazine dye, rhodamine dye, sulforhodamine dye, azo compounds, azomethine compounds, stilbene derivatives, oxazole derivatives, benzoxazole dye, imidazole dye and pyrene dye.

The amount of the organic fluorescent dye to be added to the sol solution may be appropriately adjusted depending on the type or purpose of the addition of the organic fluorescent dye. Specifically, the amount of the organic fluorescent dye to be added to the sol solution is adjusted so that the content of the organic fluorescent dye in the particles should be at least 0.001 mass%, preferably at least 0.1 mass%. Although there is no upper limit on the content of the organic fluorescent dye in the particles, it is adjusted, for example, to 10 mass% or less in the particles so as not to allow the elution of the organic fluorescent dye from the particles to be significant.

The organic fluorescent dye may be directly dissolved in the sol solution in small amounts, or a preliminary solution containing the organic fluorescent dye may be added to the sol solution. As a solvent of the preliminary solution containing the organic fluorescent dye, water may be used, or alcohols such as methanol, ethanol and isopropanol may be used. As long as the organic fluorescent dye is soluble in water or alcohol, such a preliminary solution can be prepared easily. Also for the water-soluble polymer, a preliminary solution may be prepared to be added to the sol solution in the same manner.

In order to enhance the dispersibility of the organic fluorescent dye in the sol solution, a surfactant may be added to the sol solution in addition to the organic fluorescent dye when dispersing the organic fluorescent dye in the sol solution. For example, a cationic surfactant or a glutamic acid surfactant can be used suitably. Furthermore, in order to adjust the pH of the sol solution, a pH adjuster such as sodium acetate may be added thereto.

The content of the water-soluble polymer or the organic fluorescent dye can be detected using an optical technique such as infrared absorption spectroscopy and UV-visible absorption spectroscopy. Alternatively, the content of the water-soluble polymer or the organic fluorescent dye can be calculated from the content of atoms using an elemental analysis.

### (2) Formation of particles using dye-containing sol solution

Particles are formed by spray drying process using the dye-containing sol solution that has been prepared by the above-mentioned procedure. Spray drying process is a process in which a solution is sprayed through a nozzle with a narrow hole to form microdroplets in a chamber, and then liquid is removed from the droplets in a short time. Particles are formed from the solution in a short time, thereby making the organic fluorescent dye unlikely to agglomerate in this process. As a result, particles having excellent dispersibility can be obtained. Furthermore, spray drying process is excellent in continuous production and mass production.

Spray drying process can be performed using a commercially available spray dryer. Commercially available spray dryers are procurable, for example, from PRECI Co., Ltd., fujisaki electric Co., Ltd., Powdering Japan, and Ohkawara Kakohki Co., Ltd.

The average particle size of the particles to be produced, for example, is 1 to 50 µm, though it depends also on the intended use. Particles having an average particle size in this range can be formed by appropriately adjusting the viscosity of the sol solution and the operational conditions of the spray dryer. Further, two-fluid nozzles and four-fluid nozzles using a compressed gas for spraying are provided (available from fujisaki electric Co., Ltd.) as a spraying part in a spray dryer. The use of these nozzles enables particles having an average particle size in the above-mentioned range to be formed comparatively easily. In the case where precipitates are present in the sol solution, they may be filtered before spraying.

The metal alkoxide is hydrolyzed in the sol solution, and the condensation polymerization of the hydrolysate proceeds (the condensation polymerization between two M-OH should be a dehydration condensation reaction). As a result, bonds expressed as M-O-M are formed. As the condensation polymerization proceeds, a network structure in which bonds of M-O-M extend in the form of a web is formed.

In this description, the term, average particle size, is defined as follows. That is, in the particle size distribution, a particle size that corresponds to 50% of the cumulative volume from the smaller particle size is called D50, and this value is taken as an average particle size. The particle size means a light scattering equivalent diameter of particles, as determined by a laser diffraction scattering. The light scattering equivalent diameter is defined as the diameter of a sphere that indicates a scattering pattern closest to the light scattering pattern of particles that has been obtained by the determination, and that has the same refractive index as the particles, according to "Saishin Funtai Bussei Zusetsu (Physical Properties of Powder Particles with Illustrations, Latest Version), Third edition" (issued by Yutaka KURATA, published by NGT Co., June 30, 2004), for example.

### (3) Particle drying

The particles formed by spray drying process are dried, as needed. Drying methods are not limited. However, drying may be performed by heating particles at a temperature lower than the decomposition temperature of the organic fluorescent dye in order to promote the removal of the solvent. Specifically, particles may be dried at an atmospheric temperature of 80 to 250°C.

### EXAMPLES

Hereinafter, specific examples of the present invention are shown. However, the present invention is not limited to these examples.

### Example 1

1.7 g of acetic acid was added to a solution containing 60.0 g of tetramethoxysilane, 39 g of isopropanol and 56.8 g of purified water, which was stirred at room temperature for 12 hours, so as to cause hydrolysis and polymerization. Thus, a sol solution was obtained. 0.15 g of an organic fluorescent dye (D&C Orange No.5), 3 g of polyvinylpyrrolidone (K15) and 50 g of isopropanol were added to 100 g of the sol solution, which was stirred sufficiently. Thus, a dye-containing sol solution was obtained. Using this dye-containing sol solution, particles were formed in a spray dryer (MDL-050, manufactured by fujisaki electric Co., Ltd.). The obtained particles were dried at an atmospheric temperature of 150°C for 4 hours, so that the residual solvent in the particles was removed. The obtained particles had an average particle size of 5.5 µm. For determining the average particle size, a commercially available laser diffraction particle size distribution analyzer (Microtrac HRA, manufactured by NIKKISO CO., LTD.) was used.

### Example 2

1.7 g of acetic acid was added to a solution containing 36.0 g of tetramethoxysilane, 21.4 g of methyltrimethoxysilane, 42 g of isopropanol and 56.8 g of purified water, which was stirred at room temperature for 1 hour. The resultant solution was allowed to stand still in a thermostatic device at 60°C for 24 hours, so as to cause hydrolysis and polymerization. Thus, a sol solution was obtained. 0.15 g of an organic fluorescent dye (D&C Orange No.5), 3 g of polyvinylpyrrolidone (K15) and 50 g of isopropanol were added to 100 g of the sol solution, which was stirred sufficiently. Thus, a dye-containing sol solution was obtained. Using this dye-containing sol solution, particles were formed in the same manner as in Example 1. The obtained particles were dried at an atmospheric temperature of 150°C for 4 hours. The obtained particles had an average particle size of 4.5 µm.

### Example 3

Particles were formed in the same manner as in Example 2, except that 3 g of vinyl acetate-vinylpyrrolidone copolymer was used instead of polyvinylpyrrolidone (K15). The obtained particles were dried at an atmospheric temperature of 150°C for 4 hours. The obtained particles had an average particle size of 4.3 µm.

### Example 4

Particles were formed in the same manner as in Example 2, except that 0.15 g of an organic fluorescent dye (D&C Yellow No.7) was used instead of the organic fluorescent dye (D&C Orange No.5). The obtained particles had an average particle size of 4.4 µm.

### Example 5

Particles were formed in the same manner as in Example 2, except that 0.15 g of a fluorescent whitening agent (Tinopal CBS-X, manufactured by Ciba Specialty Chemicals Inc.) was used instead of the organic fluorescent dye (D&C Orange No.5). The obtained particles had an average particle size of 5.4 µm. It should be noted that "Tinopal CBS-X" also is one of organic fluorescent dyes.

### Example 6

Particles were formed in the same manner as in Example 2, except that 0.3 g of hydroxypropyl cellulose was used instead of polyvinylpyrrolidone (K15). The obtained particles were dried at an atmospheric temperature of 150°C for 4 hours. The obtained particles had an average particle size of 7.3 µm.

### Example 7

Particles were formed in the same manner as in Example 2, except that 3 g of polyethylene glycol #1000(manufactured by NACALAI TESQUE, INC.) was used instead of polyvinylpyrrolidone (K15). The obtained particles were dried at an atmospheric temperature of 150°C for 4 hours. The obtained particles had an average particle size of 6.5 µm.

### Comparative Example 1

0.15 g of an organic fluorescent dye (D&C Orange No.5) and 50 g of isopropanol were added to the sol solution obtained in Example 1, which was stirred sufficiently. Thus, a dye-containing sol solution was obtained. Using this dye-containing sol solution, particles were formed in the same manner as in Example 1. The obtained particles had an average particle size of 5.0 µm.

### Comparative Example 2

1.7 g of acetic acid was added to a solution containing 57.0 g of tetramethoxysilane, 2.7 g of methyltrimethoxysilane, 42 g of isopropanol and 56.8 g of purified water, which was stirred at room temperature for 1 hour. The resultant solution was allowed to stand still in a thermostatic device at 25°C for 24 hours, so as to cause hydrolysis and polymerization. Thus, a sol solution was obtained. 0.15 g of an organic fluorescent dye (D&C Orange No.5) and 50 g of isopropanol were added to 100 g of this sol solution, which was stirred sufficiently. Thus, a dye-containing sol solution was obtained. Using this dye-containing sol solution, particles were formed in the same manner as in Example 1. The obtained particles were dried at an atmospheric temperature of 150°C for 4 hours. The obtained particles had an average particle size of 5.3 µm.

### Comparative Example 3

0.15 g of an organic fluorescent dye (D&C Orange No.5), 50 g of isopropanol and 1.5 g of a surfactant (Dehyquart L80, manufactured by Cognis) were added to the sol solution of Example 1, which was stirred sufficiently. Thus, a dye-containing sol solution was obtained. Using this dye-containing sol solution, particles were formed in the same manner as in Example 1. The calculated content of the surfactant was 10% as a mass ratio with respect to silica forming the porous matrix.

### <Elution resistance test>

Next, the resistance to elution of the particles in each of the examples and comparative examples were investigated by the following procedures. First, 50 mg of the particles and 10 g of water were put in a sample bottle, which was stirred with a magnetic stirrer at a rotation speed of 8.3 revolutions per second for 1 hour. This rotation speed is high enough so that the amount of elution does not change even if a higher rotation speed were employed for stirring.

Next, the particles were removed from the suspension containing the particles by suction filtration, and thereafter the intensity of the absorption peak derived from the organic fluorescent dye was measured using a visible spectrophotometer (UV-3100, manufactured by SHIMADZU CORPORATION). Then, the mass percentage of the eluted organic fluorescent dye in the organic fluorescent dye originally contained in the particles was calculated from the intensity of the absorption peak.

Further, using ethanol instead of water, the resistance to elution was examined by the same procedures. Table 1 shows the results.

### <Emission intensity>

The fluorescence of the particles in each of the examples and comparative examples were observed. The emission intensity was determined using an excitation light having a wavelength of 500 nm for the particles containing D&C Orange No.5, an excitation light having a wavelength of 480 nm for the particles containing D&C Yellow No.7 and an excitation light having a wavelength of 350 nm for the particles containing Tinopal CBS-X. Table 1 shows the results. The state of "good light emission" in Table 1 means that sufficient light emission was visually recognized when the particles were illuminated with the excitation light, even under fluorescent light. The state of "little light emission" means that light emission cannot be visually recognized therein under fluorescent light. The state of "capable of light emission" mean that slight light emission can be visually recognized therein under fluorescent light.

**Table 1**

| | Elution resistance test | | Light emission state |
|---|---|---|---|
| | Water | Ethanol | |
| EX. 1 | 3.5% | 3.2% | Good light emission |
| EX. 2 | 1.5% | 1.7% | Good light emission |
| EX. 3 | 0.7% | 1.1% | Good light emission |
| EX. 4 | 1.8% | 0.9% | Good light emission |
| EX. 5 | 2.5% | 2.2% | Good light emission |
| EX. 6 | 0.5% | 0.5% | Capable of light emission |
| EX. 7 | 1.1% | 1.2% | Good light emission |
| C. EX. 1 | over 10% | over 10% | Little light emission |
| C. EX. 2 | over 10% | over 10% | Little light emission |
| C. EX. 3 | over 10% | over 10% | Capable of light emission |

As shown in Table 1, Examples 1 to 5 and 7 exhibited good emission intensity and resistance to elution. Example 6 exhibited good resistance to elution. Comparative Examples 1 and 2 had low emission intensity, and the amount of eluted dye also was significant. Although light emission could be visually recognized in Comparative Example 3, the amount of eluted dye was significant, as in Comparative Examples 1 and 2.

The fluorescent dye-containing particle of the present invention is suitably used as a coloring agent for cosmetics because of its excellent resistance to elution and high emission intensity. The fluorescent dye-containing particle of the present invention can be used for applications such as paints, inks, resin compositions, and the like.

## Claims

1. A fluorescent dye-containing particle comprising:
a porous matrix composed mainly of a metal oxide;
an organic fluorescent dye contained in the porous matrix; and
a water-soluble polymer contained in the porous matrix.

2. The fluorescent dye-containing particle according to claim 1, wherein
the metal is silicon, and
a hydrophobic organic group is bonded to the silicon forming the porous matrix.

3. The fluorescent dye-containing particle according to claim 2, wherein
the hydrophobic organic group is at least one selected from the group consisting of alkyl group, phenyl group, alkyl halide group, vinyl group and glycidoxy group.

4. The fluorescent dye-containing particle according to claim 3, wherein
the alkyl group is at least one selected from the group consisting of methyl group, ethyl group and propyl group.

5. The fluorescent dye-containing particle according to claim 4, wherein
the alkyl group has an abundance ratio with respect to the silicon in a range of 10 to 50mo1%.

6. The fluorescent dye-containing particle according to claim 1, wherein
the water-soluble polymer is at least one selected from the group consisting of polyvinylpyrrolidone, polyvinyl alcohol, cellulose, cellulose derivatives, chitosan, acrylamide water-soluble polymer, carboxymethyl cellulose, dextrin, starch, polyethylene glycol, pectin, guar gum, xanthan gum, tamarind gum, vinyl acetate-vinylpyrrolidone copolymer, and vinylpyrrolidone-dimethylamino propyl methacrylamide copolymer.

7. The fluorescent dye-containing particle according to claim 1, wherein
the water-soluble polymer is at least one selected from the group consisting of polyvinylpyrrolidone, vinyl acetate-vinylpyrrolidone copolymer, polyethylene glycol and hydroxypropyl cellulose.

8. The fluorescent dye-containing particle according to claim 1, wherein
the water-soluble polymer has a content in a range of 1 to 30 mass%.

9. The fluorescent dye-containing particle according to claim 1, wherein
the organic fluorescent dye is soluble in water or alcohol.

10. The fluorescent dye-containing particle according to claim 1, having an average particle size in a range of 1 to 50 µm.

11. A method for producing a fluorescent dye-containing particle, comprising the steps of:
preparing a dye-containing sol solution that includes a sol solution obtained by hydrolysis of a metal compound, an organic fluorescent dye and a water-soluble polymer; and
forming a fluorescent dye-containing particle from the dye-containing sol solution by spray drying process.

12. The method for producing a fluorescent dye-containing particle according to claim 11, wherein
the metal compound to be used has a hydrophobic organic group bonded to its metal element.
